Europäisches Patentamt

European Patent Office

Office européen des brevets

(11) Veröffentlichungsnummer: **0 280 009**
**A2**

## (12) EUROPÄISCHE PATENTANMELDUNG

(21) Anmeldenummer: 88100259.6

(22) Anmeldetag: 11.01.88

(51) Int. Cl.⁴: **B01D 53/36** , B01J 21/06 ,
C07C 51/265 , C07C 63/16

(30) Priorität: 23.01.87 DE 3701984

(43) Veröffentlichungstag der Anmeldung:
31.08.88 Patentblatt 88/35

(84) Benannte Vertragsstaaten:
DE GB NL

(71) Anmelder: **Siemens Aktiengesellschaft Berlin
und München**
**Wittelsbacherplatz 2**
**D-8000 München 2(DE)**

(72) Erfinder: **Hums, Erich, Dr.**
**Aschaffenburger Strasse 16**
**D-8520 Erlangen(DE)**

(54) Verfahren zur Herstellung von Katalysatoren aus Titanoxid.

(57) Bei einem Verfahren zur Herstellung von Katalysatoren, die aus Titanoxid bestehen und über einen geeigneten Titanoxidvorläufer, wie z.B. Meta-oder Orthotitansäure hergestellt werden, soll die Zwischenstufe des Titanoxidsols vemieden werden.

Hierzu sieht die Erfindung vor, daß die Meta-oder Orthotitansäure über ein in Gegenwart eines geeigneten Flokkungsmittels bzw. Kolloids geführten hydrothermalen Prozeßschritt erhalten wird. Desweiteren kann das agglomerierte Produkt anschließend nach einem gegebenenfalls auch vor einem, eventuell auch zwischengeschalteten Formgebungsschritt calciniert werden.

Die Erfindung ist insbesondere für die Herstellung von Oxidations-und Reduktionskatalysatoren auf Titandioxidbasis anwendbar.

EP 0 280 009 A2

## Verfahren zur Herstellung von Katalysatoren aus Titanoxid

Die Erfindung bezieht sich auf ein Verfahren zur Herstellung eines Katalysators, der als Hauptkomponente Titanoxid enthält und aus einem titanoxidhaltigen Ausgangsmaterial über einen Metha- oder Orthotitansäure enthaltenden geeigneten Titanoxidvorläufer hergestellt wird.

Es ist bereits ein Verfahren zur Herstellung eines geformten Katalysators bekannt, welcher als Hauptkomponente Titanoxid enthält. Bei diesem Verfahren verwendet man ein Metha-Titansäuresol, welches durch Ausflockung von Metha-Titansäure nach dem Schwefelsäureprozeß hergestellt worden ist, indem man die Methatitansäure auf einen pH-Wert von 1 und höher eingestellt hat (DBP 26 58 569 = US-A-4,113,660). Es ist eine Eigenart dieses bekannten Herstellverfahrens für die katalytische Hauptkomponente Titanoxid, daß man das Titanoxid über ein Titanoxidsol herstellt.

Der Erfindung liegt die Aufgabe zugrunde, ein Verfahren zur Herstellung eines Katalysators zu entwickeln, bei dem man die Zwischenstufe des Titanoxidsols vermeidet.

Diese Aufgabe wird durch die Merkmale des Anspruchs 1 gelöst. Weitere, vorteilhafte Ausgestaltungen der Erfindung sind den Unteransprüchen zu entnehmen.

Wird die Ausfällung über ein Titanoxidsol entsprechend dem Stand der Technik durch einen hydrothermalen Prozeßschritt, d. h. der Agglomeration unter Druck und Temperatur ersetzt, so erhält man ein anderes, für die Verwendung als Katalysator besser geeignetes, Kristallgefüge des Titandioxids.

In Weiterbildung der Erfindung hat es sich als besonders vorteilhaft erwiesen, als Flockungsmittel lösliche Vanadiumverbindungen, lösliche Phosphate, lösliche Kieselsäureverbindungen, lösliche Molybdänverbindungen, lösliche Wolframverbindungen, Schwefelsäure oder Kohlenstoff, letzteren etwa in der Form von Graphitpulver, zu verwenden.

Weitere Ausbildungen der Erfindung werden anhand der nachfolgenden Beschreibung erläutert.

Die Herstellung eines Katalysators nach dem erfindungsgemäßen Verfahren kann beispielsweise so erfolgen, daß Meta-oder Orthotitansäure mit einem oder mehreren der obengenannten Zuschlagstoffe, z. B. mit löslichen Vanadiumverbindungen, in dem sogenannten hydrothermalen Prozeßschritt, d. h. in einem Autoklaven bei gegenüber Normalbedingungen erhöhter Temperatur, z. B. 200° C, und erhöhtem Druck. z. B. 15 bar. agglomeriert. Das so erhaltene Produkt wird nach Abdekantieren des Lösungsmittels. d. h. von Wasser, getrocknet. Nach dem Abdekantieren des Wassers kann noch ein Arbeitsgang eingeschoben werden, in dem das erhaltene Produkt vor dem Trocknen noch einmal mit Wasser gewaschen wird. um es von entsprechenden Fremdionen zu befreien. Die nach dem Trocknungsvorgang vorliegende Masse kann bedarfsweise mit Bindemitteln. wie z. B. Phosphaten, vermischt werden. Sie läßt sich anschließend nach entsprechender Formung zu Granulaten. oder nach einer Extrudierung zu Formkörpern oder nach einer Beschichtung von Platten, Streckmetallen, Sieben oder dergleichen bei Temperaturen um ca. 500° C calcinieren und dabei aushärten.

Die so erhaltenen Katalysatoren lassen sich als Reduktionskatalysatoren zur Reduktion von Stickoxiden in Gegenwart von Ammoniak einsetzen. Sie lassen sich auch beispielsweise als Oxidationskatalysatoren zur Herstellung von Phthalsäureanhydrid aus Orthoxylol oder Maleinsäureanhydrid aus $C_4$-Kohlenwasserstoffen verwenden.

In Fällen, in denen das Katalysatormaterial in rutilisierter Form (Rutil = bestimmte Kristallkonfigurationdes Titanoxids) vorliegen soll, sind Rutilisierungsbildner - das sind beispielsweise Lithium-, Kaliumsalze oder CuO der Metha-und Orthotitansäure - vor Anfahren des hydrothermalen Prozeßschrittes der Ausgangssubstanz zuzusetzen.

Ausführungsbeispiel:

Zu dem Ausgangsmaterial Metha-oder Orthotitansäure wird ein Flockungsmittel nach Art und Menge zudosiert. So zum Beispiel werden ein bis zwei Gewichtsprozent Orthokieselsäure oder deren Derivate der wäßrigen Suspension von Metha-oder Orthotitansäure zugemischt, wobei sich ein pH-Wert im Bereich von schwach sauer bis neutral einstellt. Diese Mischung wird in einem sich anschließenden hydrothermalen Prozeßschritt agglomeriert. Dieser hydrothermale Prozeßschritt erfolgt vorzugsweise in einem Autoklaven bei Temperaturen von 180° C und dem entsprechenden sich bei dieser Temperatur einstellenden Dampfdruck des Wassers. Je nach Wahl der Temperatur kann die Behandlungsdauer in dem Autoklaven zwischen Stunden (bei 180°) und Tagen (bei entsprechend niedriger Temperatur) dauern. Durch diesen Prozeßschritt wird Einfluß auf die Bildung der Primärkorngrößen - d. h. der Größe der sich bildenden Mikrokristallite - und Sekundärkorngrößen - d. h. der Größe der Agglomerate der Mikrokristallite - genommen. Die Primärkorngröße liegt bei 10 bis 30 nm (Nannometer) und die Sekundärkorngröße liegt bei 2 bis 20 µm. Das erhaltene Produkt wird durch Filtration von Wasser befreit, anschließend getrocknet und danach bei ca.

300° bis 500° C calciniert.

Werden anstelle von der eingangs genannten Kieselsäure andere Flockungsmittel, wie z. B. Phosphor in Form von Phosphorsäure oder Molybdänverbindungen in Form von Ammoniummolybdat, eingesetzt, so stellen sich daraus folgend in der wäßrigen Suspension entsprechend der Stärke der verwendeten Säure andere pH-Werte ein. Als Flockungsmittel eignen sich besonders gut Am moniumvanadat, Ammoniummolybdat, Ammoniumwolframat, Phosphorsäure, Schwefelsäure, Graphitpulver und lösliche Kieselsäureverbindungen. Bei der Verwendung von Kieselsäure, Molybdänverbindungen und Wolframverbindungen wird zugleich der Anteil des Titanoxids, der in Rutilform vorliegt, gegenüber jenem der in der Anastasform vorliegt, vermehrt.

Ansprüche

1. Verfahren zur Herstellung eines Katalysators, der als Hauptbestandteil Titanoxid enthält und aus einem titanoxidhaltigen Ausgangsmaterial über einen geeigneten Metha-oder Orthotitansäure enthaltenden Titanoxidvorläufer hergestellt wird, **dadurch gekennzeichnet,** daß die Metha-oder Orthotitansäure über einen in Gegenwart eines geeigneten Flokkungsmittels oder Kolloids geführten hydrothermalen Prozeßschritt erhalten wird.

2. Verfahren nach Anspruch 1, **dadurch gekennzeichnet,** daß als Flockungsmittel lösliche Vanadiumverbindungen, vorzugsweise Ammoniumvandat, verwendet werden.

3. Verfahren nach Anspruch 1, **dadurch gekennzeichnet,** daß als Flockungsmittel lösliche Phosphate, vorzugsweise Phosphorsäure, verwendet werden.

4. Verfahren nach Anspruch 1, **dadurch gekennzeichnet,** daß als Flockungsmittel lösliche Kieselsäureverbindungen verwendet werden.

5. Verfahren nach Anspruch 1, **dadurch gekennzeichnet,** daß als Flockungsmittel Kohlenstoff, beispielsweise in Form von Graphitpulver, verwendet wird.

6. Verfahren nach Anspruch 1, **dadurch gekennzeichnet,** daß als Flockungsmittel lösliche Molybdänverbindungen, vorzugsweise in Form von Ammoniummolybdat, verwendet werden.

7. Verfahren nach Anspruch 1, **dadurch gekennzeichnet,** daß als Flockungsmittel lösliche Wolframverbindungen, vorzugsweise Ammoniumwolframat, verwendet werden.

8. Verfahren nach Anspruch 1, **dadurch gekennzeichnet,** daß als Flockungsmittel Schwefelsäure verwendet wird.

9. Verfahren nach Anspruch 1, **dadurch gekennzeichnet,** daß das agglomerierte Produkt anschließend nach einem, gegebenenfalls auch vor einem, eventuell zwischengeschalteten Formgebungsschritt calciniert wird.

10. Verfahren nach Anspruch 1, **dadurch gekennzeichnet,** daß das Verfahren zur Titanoxidherstellung in Richtung vermehrter Rutilbildung durch entsprechende Zugaben von Rutilisierungsbildnern verschiebbar ist.

11. Verfahren nach Anspruch 1, **dadurch gekennzeichnet,** daß das Produkt als Reduktionskatalysator zur Reduktion von Stickoxiden in Gegenwart von Reduktionsmitteln, wie z. B. $NH_3$, CO oder Kohlenwasserstoffe, eingesetzt wird.

12. Verfahren nach Anspruch 1, **dadurch gekennzeichnet,** daß das Produkt als Oxidationskatalysator beispielsweise zur Herstellung von Phthalsäureanhydrid aus Orthoxylol eingesetzt wird.